(19) Europäisches Patentamt
European Patent Office
Office européen des brevets

(11) Publication number: **0 109 294**
**A2**

(12) # EUROPEAN PATENT APPLICATION

(21) Application number: **83306923.0**

(22) Date of filing: **11.11.83**

(51) Int. Cl.³: **C 07 D 501/36**, A 61 K 31/545

(30) Priority: **15.11.82 JP 200898/82**

(43) Date of publication of application: **23.05.84**
**Bulletin 84/21**

(84) Designated Contracting States: **BE CH DE FR GB IT LI NL SE**

(71) Applicant: **Takeda Chemical Industries, Ltd., 27, Doshomachi 2-chome Higashi-ku, Osaka-shi Osaka, 541 (JP)**

(72) Inventor: **Yoshimura, Yoshinobu, 1-406, Tarumicno 2-chome, Suita Osaka 564 (JP)**
Inventor: **Hamaguchi, Naoru, 6-704, 2 Shirakawa 3-chome, Ibaraki Osaka 567 (JP)**
Inventor: **Yashiki, Takatsuka, 20-18, Izumigaoka, Takarazuka Hyogo 665 (JP)**

(74) Representative: **Myerscough, Philip Boyd et al, J.A.Kemp & Co. 14, South Square Gray's Inn, London, WC1R 5EU (GB)**

(54) **Cephalosporin esters.**

(57) A compound of the formula

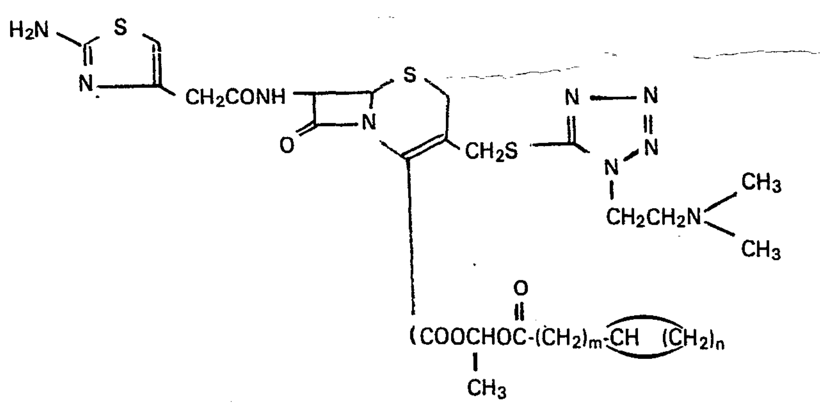

wherein m is an integer of 0 or 1; n is an integer of 2 to 5, inclusive, or its pharmaceutically acceptable salt, which is effective as orally administrable antibiotic agents against both gram-positive- and -negative-bacteria, and the production and compositions thereof, are proposed.

EP 0 109 294 A2

0109294

# CEPHALOSPORIN ESTERS

This invention relates to a compound of the formula:

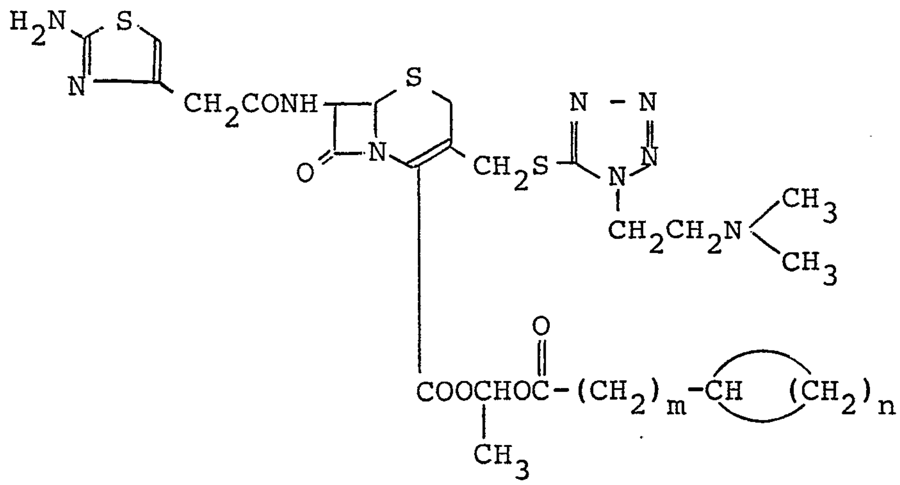

(I)

[wherein m is an integer of 0 or 1; n is an integer of 2 to 5, inclusive] or a pharmaceutically acceptable salt thereof, particularly an acid addition salt and to a process for producing the same. It also relates to a pharmaceutical composition and an antibiotic use thereof.

For the purpose of increasing the absorption into the body after oral administration of the non-ester form of compound (I), i.e. 7β-[2-(2-aminothiazol-4-yl)-acetamido]-3-[[[1-(2-dimethylaminoethyl)-1H-tetrazol-5-yl]thio]methyl]ceph-3-em-4-carboxylic acid (which is hereinafter referred to briefly as compound (II); U.S. Patent No. 4080498), it has been proposed to convert (II) into a straight-chain or branched alkylcarbonyloxyalkyl ester such as the pivaloyloxymethyl ester, (U.S. Patent No. 4189479 and Japanese Published Unexamined Patent Application No. 77690/1982 published on May 15, 1982). However, a further improvement has been demanded in the aspects of absorption, stability, etc.

The present inventors have found through investigation of a series of ester derivatives of compound (II), that the compound of formula (I) or an acid addition salt thereof can be absorbed well through the gastrointestinal tract until the unesterified form of compound (I) is liberated soon after the absorption and is well transferred into the circulation to produce a high blood level of compound (II). They have also found that the compound (I) is effective as an orally administrable broad-spectrum antibiotic having activity not only against gram-positive and gram-negative bacteria but also active against resistant strains thereof. It is also found that conversion of this compound (I) to an acid addition salt increases the water solubility of the ester to thereby improve its rate of absorption, stabilizes compound (I) and facilitates both the isolation of compound (I) and the pharmaceutical processing thereof.

Referring to the above cephalosporin compound (I) according to the present invention, the group represented by the formula:

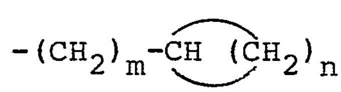

[wherein m and n are as defined hereinbefore] at the carboxy ester moiety in 4-position of the cephalosporin nucleus is a cycloalkyl group containing 3 to 6 carbon atoms such as cyclopropyl, cyclobutyl, cyclopentyl, cyclohexyl, etc. or a cycloalkylmethyl group of 4 to 7 carbon atoms such as cyclopropylmethyl, cyclobutylmethyl, cyclopentylmethyl, cyclohexylmethyl, etc. Preferred species are cyclopentyl, cyclohexyl, cyclopentylmethyl and cyclohexylmethyl, with cyclopentylmethyl and cyclohexylmethyl being the most preferred.

Since compound (I) is basic in itself, it can be converted to acid addition salts. Generally, one

molar equivalent of compound (I) forms an acid addition salt with 1 or 2 molar equivalents of an acid. Preferred examples of the acid that can be used for the formation of such acid addition salt include acids which are generally known to provide pharmaceutically acceptable salts in the field of penicillins and cephalosporins. Thus, for example, such inorganic acids as hydrochloric acid, sulfuric acid, phosphoric acid, etc. and such organic acids as maleic acid, acetic acid, citric acid, succinic acid, tartaric acid, malic acid, malonic acid, fumaric acid, benzoic acid, mandelic acid, ascorbic acid, methanesulfonic acid, etc. may be mentioned.

The acid addition salt of compound (I) is preferably the monohydrochloride or dihydrochloride, and preferably the dihydrochloride. The aminothiazole group of compound (I) or acid addition salt thereof may exist as an iminothiazoline group which is a tautomer thereof. Since the compound (I) or acid addition salt thereof contains an asymmetric carbon atom in the 4-carboxy ester moiety of the cephalosporin nucleus, it may exist as two optical isomers (D- and L-isomers). Therefore, the compound (I) or acid addition salt thereof may be used optionally in the form of a racemic compound, either the D-isomer or the L-isomer, or a mixture of optional proportions of such isomers. The compound (I) and acid addition salt thereof are absorbed well from the gastrointestinal tract and, upon absorption, its 4-carboxy ester moiety is rapidly hydrolyzed by the enzymes in the body to give the non-ester form of compound (I), that is compound (II).

As described in Antimicrobial Agent and Chemotherapy 14, 557-568 (1978), this compound (II) has strong antibiotic activity.

Thus, the compound (II) is highly active against gram-positive bacteria such as Staphylococcus aureus, etc. and gram-negative bacteria such as Escherichia coli, Klebsiella pneumoniae, Proteus vulgaris, Proteus mirabilis and Proteus morganii.

When administered by the oral route, the compound (I) yields a high concentration in blood and it is effective for the treatment of infections in human and other mammalia caused by said bacteria, for example respiratory or urinary infections.

The compound (I) or an acid addition salt thereof according to the present invention is low in toxicity ($LD_{50}$ ≧ 3 g/kg, mice, oral) and can orally be administered, and can be formulated into capsules, powders, fine granules, granules or tablets, etc., in admixture with the common pharmaceutically acceptable excipients e.g. starch, lactose, calcium carbonate, calcium phosphate, etc., binders e.g. starch, gum arabic, carboxymethylcellulose, hydroxypropylcellulose, crystalline cellulose, etc., lubricants e.g. magnesium stearate, talc, etc., or/and disintegrators e.g. carboxymethylcellulose calcium, talc, etc., in accordance with the established pharmaceutical procedure. Granules can also be produced by blending the compound (I) or acid addition salt thereof with about 1 to 5 molar equivalents of a solid organic acid, e.g. citric acid, malic acid, tartaric acid, succinic acid, ascorbic acid, mandelic acid, etc. Such granules can be further processed into capsules, tablets, etc. by the established pharmaceutical procedure.

The recommended dosage of compound (I) or acid addition salt thereof is 0.3 to 5 grams and preferably 0.5 to 3 grams per day per adult human, given in 3 to 4 divided doses.

The compound (I) or acid addition salt thereof can be produced by known methods <u>per se</u> i.e. the processes described in U.S. Patents 4080498 and 4189479 and Japanese Published Unexamined Patent Application No. 77690/82, for instance. The compound (I) or a salt thereof can also be produced by esterifying compound (II) or a salt thereof with a compound of the formula:

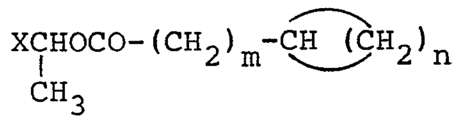

(III)

[wherein X is a halogen atom; m and n are as defined hereinbefore].

Referring to the above formula (III), the halogen X may for example be chlorine, bromine or iodine.

Of such compounds (III), one (III) wherein X is iodine, i.e. an iodoethyl acylate, is preferably employed in the above esterification reaction.

Since the compound (III) contains an asymmetric carbon atom, it can be submitted to the reaction after resolving it into the D- and L-isomers, thus, in the form of either the D-isomer or the L-isomer, or as an optional mixture of such isomers. The starting compound (II) for the present invention can be used in the contemplated reaction after converting it to its acid addition salt with an inorganic acid, e.g. hydrochloric acid, sulfuric acid, nitric acid, etc. or an organic acid, e.g. oxalic acid, p-toluenesulfonic acid, etc. or its salt with a base such as alkali metals, e.g. sodium, potassium, etc., alkaline earth metals, e.g. calcium, magnesium, etc. or an organic amine, e.g. triethylamine, trimethylamine, pyridine, collidine, lutidine, etc.

For the above-mentioned esterification reaction, the starting compound (III) is used in a proportion of about 1 to 10 molar equivalents, preferably 1 to 1.5 molar equivalen to compound (II) or a salt thereof. This esterification reaction is generally conducted in a solvent inert to the reaction. As examples of said solvent may be mentioned amides such as N,N-dimethylformamide (hereinafter referred to as DMF), N,N-dimethylacetamide (hereinafter referred to as DMAC), hexamethylphosphorotriamide (hereinafter referred to as HMPA) etc., halogenated hydrocarbons such as dichloromethane, chloroform, etc., sulfoxides such as dimethylsulfoxide (hereinafter referred to as DMSO), sulfolane, etc., ethers such as dioxane, tetrahydrofuran (hereinafter referred to as THF), etc., ketones such as acetone, methyl ethyl ketone etc., nitriles such as acetonitrile, etc., and anhydrous liquefied sulfur dioxide. Preferred species among these solvents are DMF, DMAC, HMPA, acetone, acetonitrile, anhydrous liquefied sulfur dioxide. This esterification reaction is conducted generally at a temperature of -20 to 20°C. A catalyst is not always needed. It may, however, be conducted in the presence of a catalyst such as a phase transfer catalyst, e.g. 18-crown-6. When anhydrous liquefied sulfur dioxide is used as the solvent, the reaction is desirably conducted at a temperature near the boiling point of the solvent, i.e. -10 to -20°C. While the reaction time varies with different species of reactants and types of solvents, it is generally about 10 minutes to about 6 hours.

The compound (I) or an acid addition salt thereof can also be produced by the following process, for instance.

A compound of formula:

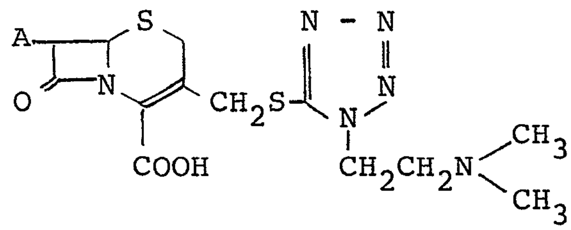

(IV)

[wherein A is an amino group or an acylamino group other than 2-(2-aminothiazol-4-yl)acetylamino] is reacted with a compound of formula (III) in the same manner as the above-mentioned esterification reaction. When A is an acylamino group, the resulting ester is further reacted with phosphorous pentachloride and, then, with an alcohol, e.g. methanol, ethanol, propanol, isopropyl alcohol, n-butanol, etc. [cf. Journal of Medicinal Chemistry 18, 992 (1975), and German Laid-open Patent Applications (Offenlegungsschriften) Nos. 2460331 and 2460332). The resulting compound, which can be represented by the formula:

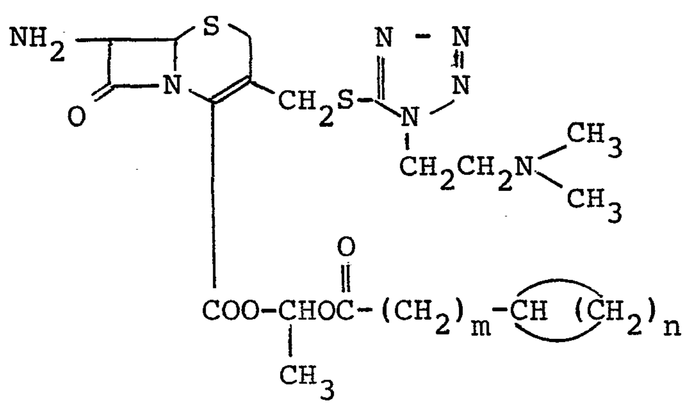

(V)

[wherein the symbols have the same meanings as defined hereinbefore] is acylated with the compound of the formula:

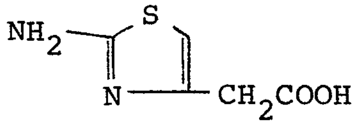

(VI)

which is 2-(2-aminothiazol-4-yl)acetic acid.

Referring to the above formula (IV) wherein A is an acylamino group, the acyl group may be any such group that is commonly used in the field of cephalosporin compounds. Preferred examples of the acylamino group include acetylamino, benzoylamino, phenylacetylamino, thienylacetylamino, phenyloxyacetylamino and 5-amino-5-carboxyvalerylamido. The amino group may optionally be protected with phthaloyl or the like. When A is an amino group or an amino-substituted acylamino group, these amino groups are preferably protected. Protective groups that can be used for this protection include, among others, t-butoxycarbonyl, carboxybenzyloxy, 2-hydroxy-1-naphthocarbonyl, trichloroethoxycarbonyl, 2-ethoxycarbonyl-1-methylvinyl and 2-methoxycarbonyl-1-methylvinyl.

Deacylation of the ester compound produced by reacting compound (IV), wherein A is an acylamino group, with compound (III) is carried out in a per se known manner. Generally, about 2 to 5 molar equivalents of phosphorous pentachloride and about 10 to 40 molar equivalents of alcohol are used for each molar equivalent of the material ester compound. This reaction is generally conducted in an inert solvent such as halogenated hydrocarbons, e.g. dichloromethane, chloroform, etc. For the purpose of accelerating the reaction, there may be added a tertiary amine such as triethylamine, pyridine, N,N-dimethylaniline, etc. The reaction is carried out within the temperature range of about -40 to 20°C. A reaction time of 1 hour is generally sufficient.

In reacting the resulting compound (V) with compound (VI) to produce compound (I) or an acid addition salt thereof, the amino group of compound (VI) is preferably protected. Protective groups may be the same as those mentioned for the protection of the amino group of

compound (IV). In conducting this acylation reaction, compound (VI) may be used in the form of a reactive derivative such as the corresponding acid halide, acid anhydride, mixed acid anhydride, active amide, activated ester, etc.

Examples of said activated ester include p-nitrophenyl ester, 2,4-dinitrophenyl ester, pentachlorophenyl ester, N-hydroxyphthalimide ester, etc. The mixed acid anhydride mentioned above includes, for example, mixed acid anhydrides with carbonic acid monoesters such as carbonic acid monomethyl ester, carbonic acid monoisobutyl ester, etc. and mixed acid anhydrides with lower or 2 to 5 carbon atoms alkanoic acids which may optionally be substituted by halogen, such as pivalic acid, trichloroacetic acid, etc.

When compound (VI) is used is the form of a free compound or a salt, a suitable condensing agent is employed. Examples of such condensing agent include dehydrating agents such as N,N'-disubstituted carbodiimides, e.g. N,N'-dicyclohexylcarbodiimide, etc., azolides, e.g. N,N'-carbonylimidazole, N,N'-thienyldiimidazole, etc., N-ethoxycarbonyl-2-ethoxy-1,2-dihydroquinoline, phosphorous oxychloride, alkoxyacetylene, e.g. ethoxyacetylene, etc., etc. It is considered that when such a condensing agent is employed, the reaction proceeds via formation of a reactive derivative at the carboxyl function.

This reaction can generally be conducted in a solvent. As the solvent, there may be employed any of the common solvents, as well as a suitable mixture thereof, which does not interfere with the reaction, such as water, acetone, diisobutyl ketone, THF, ethyl acetate, dioxane, acetonitrile, chloroform, dichloromethane, dichloroethylene, pyridine, dimethylaniline, DMF, DMAC, DMSO, etc.

The reaction temperature is virtually optional but the reaction is generally conducted under cooling or at room temperature. When the reaction proceeds with liberation of acid, a base is added to the reaction system as required. The base mentioned above is exemplified by aliphatic, aromatic or heterocyclic-nitrogen bases, alkali metal carbonates and alkali metal hydrogen carbonates, such as triethylamine, N,N-dimethylaniline, N-ethylmorpholine, pyridine, collidine, 2.6-lutidine, sodium carbonate, potassium carbonate, potassium hydrogen carbonate, sodium hydrogen carbonate, etc. When the acylation reaction is predominantly a dehydrative reaction, water is preferably eliminated from the solvent. In some cases, the reaction may be carried out under anhydrous conditions in an inert gas such as nitrogen gas. When the reaction product has a protective group, the protective group is eliminated by a *per se* known procedure.

Compound (I) or an acid addition salt thereof can also be produced by reacting compound (V) with a 4-halo-3-oxobutyryl halide, which can be obtained by reacting diketene with a halogen e.g. chlorine, bromine, iodine, in an equimolar ratio, to give a compound of formula (VII):

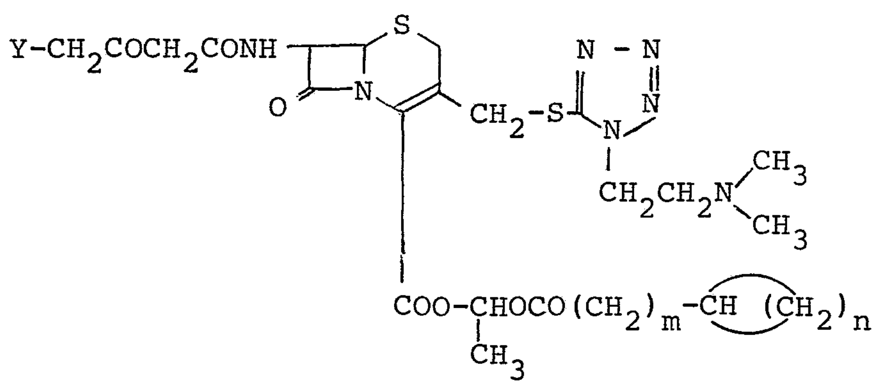

(VII)

[wherein m and n are as defined hereinbefore; Y is a halogen atom, e.g. chlorine, bromine, iodine] and reacting compound (VII) further with thiourea. In this reaction of compound (VII) with thiourea, the latter may be used either as such or in such other forms as salts with alkali metals such as lithium, sodium, potassium, etc., ammonium salts, etc. The reaction is generally conducted by admixing and reacting the two reactants in an equimolar ratio in a solvent and, if necessary, in the presence of 1 to 2 molar equivalents of a base. The solvent suitable for this reaction includes water, methanol, ethanol, acetone, dioxane, acetonitrile, chloroform, ethylene chloride, THF, ethyl acetate, DMF, DMAC, DMSO, etc. Among such solvents, hydrophilic solvents can be used in admixture with water. The base mentioned above includes, among others, alkali metal hydroxides such as sodium hydroxide, potassium hydroxide, etc., alkali metal carbonates such as sodium carbonate, potassium carbonate, etc., alkali metal hydrogen carbonates such as sodium hydrogen carbonate, and organic tertiary amines such as triethylamine, trimethylamine, pyridine, etc. There is virtually no limitation on the reaction temperature but generally the reaction is preferably conducted under cooling. The reaction proceeds fast and generally goes to completion within 10 minutes, although a reaction time of as long as 30 minutes or more may be required in some cases. The compound (VII) can be easily produced by the above process or *per se* known procedures.

Furthermore, the compound (I) or its pharmaceutically acceptable salt is prepared by the following steps:

(1) A compound of the formula:

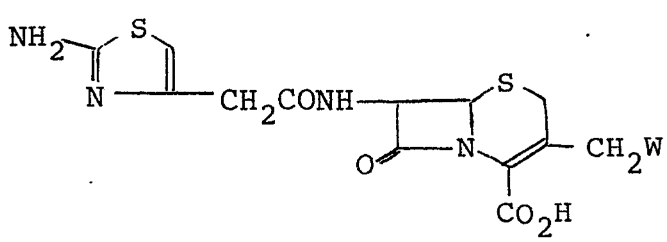

(VIII)

[wherein W is an acetoxy or an acetoacetoxy group], is allowed to react with a compound (III) under similar conditions of esterification to those described above, thereby to produce a compound of the formula (IX) below

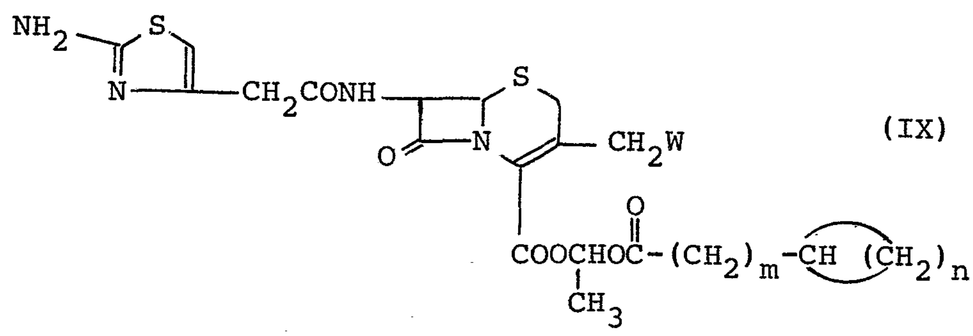

(IX)

[wherein m, n and W have the same meanings as defined hereinbefore], and (2) then the compound is allowed to react with 1-(2-dimethylaminoethyl)-5-mercapto-1H-tetrazole.

In the reaction (2), 1-(2-dimethylaminoethyl)-5-mercapto-1H-tetrazole is used in a proportion of about 1 to 3 molar equivalents relative to compound (IX).

This reaction (2) can be usually carried out in a solvent inert to the reaction. Any conventional solvent such as water, THF, ethyl acetate, dioxane, acetonitrile, chloroform, dichloromethane, DMF, DMAC, DMSO and a mixture thereof can be used.

In the case of water, another water missible solvent is recommended to admix. Presence of an inorganic base in the reaction system is preferable. Examples of the base are alkali metal carbonate e.g. sodium carbonate and potassium carbonate, alkali metal bicarbonate e.g. sodium bicarbonate, potassium bicarbonate, alkaline earth metal carbonate e.g. calcium carbonate and so on. Amount of the base present in the reaction is

about 1 to 3 moles to a co-reactant of the compound (IX). The reaction temperature range is not particularly limited, but normally room temperature or about 15°C to 60°C, and the reaction time is roughly from 30 minutes to 3 hours, depending on kinds of solvents and temperature.

Referring to the above reactions, when the objective compound (I) or an acid addition salt thereof includes the $\Delta^2$-isomer, the latter can be isomerized to the $\Delta^3$-isomer by a per se known procedure, for example the process described in Journal of Medicinal Chemistry 18, 986 (1975), or the entirety is first converted to the corresponding S-oxide and the resulting $\Delta^3$-isomer is then reduced to the desired compound (I) or a salt thereof.

When the objective compound (I) is produced in a free form, it can be converted to a desired acid addition salt thereof by dissolving the compound (I) in a free form in an inert solvent such as dichloromethane, chloroform or the like and adding about 1 to 10 molar equivalents of an acid. When compound (I) or an acid addition salt thereof is obtained as a racemic isomer, it can be resolved into optically active compounds (D- and L-isomers) by a per se known procedure. The resulting compound (I) or an acid addition salt thereof can be isolated and purified by a per se known procedures such as solvent extraction, pH adjustment, phase transfer, crystallization, recrystallization, chromatography, etc.

The starting compound (III) can be produced by a procedure known per se. Moreover, compound (III) can

also be produced by the procedures schematically illustrated below.

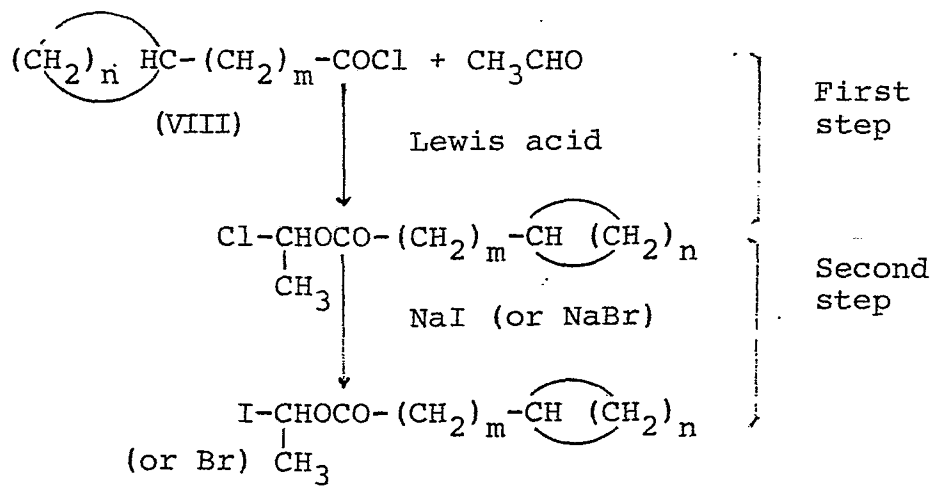


In the above formulas, m and n are as defined hereinbefore.

Referring to the formula (III), the compound wherein X is iodine, i.e. an iodoethyl acylate, can be produced by reacting the acid chloride (VIII) with paraldehyde or acetaldehyde in the presence of a Lewis acid and reacting the resulting chloroethyl acylate with sodium iodide. The first step reaction is conducted in the presence of a Lewis acid catalyst such as anhydrous zinc chloride, aluminum chloride, stannic chloride, etc. This reaction proceeds under cooling at about -40 to +30°C, preferably at about -40 to 0°C, or under heating at about 30 to 140°C, preferably at about 90 to 140°C. While the reaction time varies with the reaction temperature used, it is about 1 to 3 hours under cooling and about 1 to 6 hours under heating. The reaction proceeds satisfactorily even in the absence of a solvent.

After completion of the first step reaction, the reaction mixture is subjected to distillation, column chromatography or the like to recover the chloroethyl acylate. This chloroethyl acylate is then reacted with

sodium iodide to give the iodoethyl acylate (the second step reaction). This second step reaction is conducted in the common solvent, e.g. acetone, acetonitrile, DMF, DMSO, etc. The reaction temperature may be as mild as room temperature to about 40 to 50°C. The reaction time is about 15 minutes to 6 hours, preferably about 15 minutes to 2 hours.

The reaction product can be isolated and purified, for example, by solvent extraction, pH adjustment, distillation, distillation under reduced pressure, phase transfer, chromatography, etc.

The following reference examples, working examples and test example are further illustrative but by no means limitative of the present invention.

The symbols used in these examples have the following meanings.

s: singlet
b-s: broad singlet
d: doublet
d.d: double doublet
t: triplet
q: quartet
ABq: AB-pattern quartet
m: multiplet
bp: boiling point

Reference Example 1

1-Iodoethyl cyclopropanecarboxylate

a) To 25 g of cyclopropanecarbonyl chloride is added a catalytic amount of anhydrous zinc chloride, followed by cooling to -20°C. Under stirring, 15 ml of acetaldehyde is added dropwise and the mixture is further stirred at the same temperature for 1 hour. The reaction

mixture is subjected to silica gel chromatography using petroleum ether as the eluent. The eluate is collected and the solvent is distilled off under reduced pressure, followed by further distillation under reduced pressure to give 22 g of 1-chloroethyl cyclopropylcarboxylate as a colorless oil.

bp 81-82°C/35-37 mmHg
IR(liquid film) $cm^{-1}$: 1760, 1750, 670
NMR ($CDCl_3$) δ: 0.8-1.2(m, 4H), 1.4-1.9(m, 1H), 1.79(d, J = 6Hz, 3H), 6.56(q, J = 6Hz, 1H)
Elemental analysis for $C_6H_9O_2Cl$
Calcd.: C, 48.50; H, 6.10 (%)
Found : C, 48.25; H, 6.27 (%)

b) Acetonitrile (100 ml) is warmed to 40°C and 16 g of sodium iodide is dissolved therein. To this solution is added 8 g of the 1-chloroethyl cyclopropanecarboxylate prepared above and the mixture is stirred for 15 minutes. The insolubles are filtered off and the resulting filtrate is concentrated under reduced pressure. To the residue is added a mixture of petroleum ether and 5% aqueous sodium thiosulfate and the petroleum ether layer is separated. The organic layer is washed with 5% aqueous sodium thiosulfate and dried over anhydrous magnesium sulfate, followed by evaporation of the solvent under reduced pressure to recover 5.3 g of the above-identified compound.

IR(liquid film) $cm^{-1}$: 1755, 1750

The compounds produced by the same procedure as Reference Example 1 a) and their physical and chemical properties are shown below.

1-Chloroethyl cyclobutanecarboxylate

bp 90-92°C/40-42 mmHg

IR(liquid film) $cm^{-1}$: 1770, 1755, 670

NMR ($CDCl_3$) δ: 1.75 (d, J=6Hz, 3H), 1.7-2.6 (m, 6H), 2.9-3.4 (m, 1H), 6.54 (q, J=6Hz, 1H)

Elemental analysis for $C_7H_{11}O_2Cl$

Calcd.: C, 51.70; H, 6.82 (%)

Found : C, 51.59; H, 6.74 (%)

1-Chloroethyl cyclopentanecarboxylate

bp 70-72°C/4-5 mmHg

IR(liquid film) $cm^{-1}$: 1760, 1740, 670

1-Chloroethyl cyclopentylacetate

bp 64-65°C/3-4 mmHg

IR(liquid film) $cm^{-1}$: 1765, 1755, 670

NMR ($CDCl_3$) δ: 1.0-2.1 (m, 9H), 1.77 (d, J=6Hz, 3H), 2.2-2.5 (m, 2H), 6.55 (q, J=6Hz, 1H)

Elemental analysis for $C_9H_{15}O_2Cl$

Calcd.: C, 56.69; H, 7.93 (%)

Found : C, 57.26; H, 7.95 (%)

1-Chloroethyl cyclohexanecarboxylate

bp 70-72°C/4 mmHg

IR(liquid film) $cm^{-1}$: 1765, 1750, 670

NMR ($CDCl_3$) δ: 0.8-2.15 (m, 10H), 1.77 (d, J=6Hz, 3H), 2.15-2.60 (m, 1H), 6.55 (q, J=6Hz, 1H)

Elemental analysis for $C_9H_{15}O_2Cl$

Calcd.: C, 56.69; H, 7.93 (%)

Found : C, 56.93; H, 7.92 (%)

1-Chloroethyl cyclohexylacetate

bp 77-79°C/4 mmHg

IR(liquid film) $cm^{-1}$: 1765, 1755, 670

NMR ($CDCl_3$) δ: 0.8-2.2 (m, 11H), 1.78 (d, J=6Hz, 3H), 2.2-2.5 (m, 2H), 6.54 (q, J=6Hz, 1H)

Elemental analysis for $C_{10}H_{17}O_2Cl$
Calcd.: C, 58.68; H, 8.37 (%)
Found : C, 58.43; H, 8.12 (%)

The compounds produced by the same procedure as Reference Example 1 b) and their physico-chemical properties are shown below.

1-Iodoethyl cyclobutanecarboxylate
IR(liquid film) $cm^{-1}$: 1765, 1755

1-Iodoethyl cyclopentanecarboxylate
IR(liquid film) $cm^{-1}$: 1760, 1740

1-Iodoethyl cyclopentylacetate
IR(liquid film) $cm^{-1}$: 1760, 1750

1-Iodoethyl cyclohexanecarboxylate
IR(liquid film) $cm^{-1}$: 1760, 1750

1-Iodoethyl cyclohexylacetate
IR(liquid film) $cm^{-1}$: 1760, 1755

Example 1

1-(Cyclohexylacetoxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate (Compound No. 1)

In 50 ml of dimethylformamide is dissolved 5.22 g of sodium 7β-[2-(2-aminothiazol-4-yl)acetamido]-3-[[[1-(2-dimethylaminoethyl)-1H-tetrazol-5-yl]thio]methyl]-

ceph-3-em-4-carboxylate and the solution is cooled to -5°C. Under stirring, to it 10 g of 1-iodoethyl cyclohexyl-acetate is added at one stroke and the mixture is kept under stirring for 5 minutes. The reaction mixture is then poured in a mixture of 300 ml ethyl acetate and 300 ml ice-water and the organic layer is separated. The aqueous layer is extracted with 200 ml of ethyl acetate and the organic layers are combined and washed 3 times with 300 ml portions of ice-water and once with 300 ml of saturated aqueous sodium chloride, followed by drying over anhydrous magnesium sulfate. The solvent is distilled off under reduced pressure and isopropyl ether is added to the residue. The resulting powder is collected by filtration and recrystallized from acetone-isopropyl ether to give 3.9 g of the above-identified compound as colorless crystals.

IR(KBr) $cm^{-1}$: 1775, 1750, 1730, 1670

NMR ($d_6$-DMSO) δ: 1.02 (d, J=6Hz, 2H), 0.77-1.93 (m, 11H), 1.47 and 1.50 (two d, J=6Hz, 3H), 2.22 (s, 6H), 2.75 (t, J=6Hz, 2H), 3.37 (s, 2H), 3.57 and 3.83 (ABq, J=18Hz, 2H), 4.01 and 4.16 (ABq, J=13.5Hz, 2H), 4.40 (t, J=6Hz, 2H), 5.05 (d, J=4.5Hz, 1H), 5.56 - 5.95 (m, 1H), 6.23 (s, 1H), 6.82 (s, 2H), 6.66-7.12 (m, J=6Hz, 1H) and 8.85 (d, J=9Hz, 1H)

Elemental analysis for $C_{28}H_{39}N_9O_6S_3 \cdot 1/2H_2O$

Calcd.: C, 47.83; H, 5.75; N, 17.96 (%)

Found : C, 48.19; H, 5.70; N, 17.44 (%)

The compounds produced by the same procedure as Example 1 and their physico-chemical constants are as follows.

1-(Cyclohexanecarbonyloxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate (Compound No. 3)

IR(KBr) $cm^{-1}$: 1775, 1740, 1638

NMR ($d_6$-DMSO) δ: 1.43 and 1.47 (two d, J=6Hz, 3H), 0.93-2.0 (m, 10H), 2.20-2.42 (m, 1H), 2.17 (s, 6H), 2.67 (t, J=6Hz, 2H), 3.37 (s, 2H), 3.57 and 3.82 (ABq, J=18Hz, 2H), 3.99 and 4.17 (ABq, J=13.5Hz, 2H), 4.36 (t, J=6Hz, 2H), 5.07 (d, J=4.5Hz, 1H), 5.69 (d.d, J=4.5Hz and 8.7Hz, 1H), 6.21 (s, 1H), 6.81 (s, 2H), 6.93-7.12 (m, 1H) and 8.84 (d, J=8.7Hz, 1H)

Elemental analysis for $C_{27}H_{37}N_9O_6S_3$

Calcd.: C, 47.70; H, 5.49; N, 18.54 (%)

Found : C, 47.39; H, 5.42; N, 18.13 (%)

1-(Cyclopentylacetoxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate (Compound No. 5)

IR(KBr) $cm^{-1}$: 1774, 1750, 1635

NMR ($d_6$-DMSO) δ: 1.47 and 1.55 (two d, J=6Hz, 2H), 0.90-2.0 (m, 9H), 2.19 (s, 6H), 2.27 and 2.34 (two d, J=6Hz, 1H) 2.70 (t, J=6Hz, 2H), 3.33 (s, 2H), 3.53 and 3.83 (ABq, J=18Hz, 2H), 4.03 and 4.13 (ABq, J=13.5Hz, 2H), 4.38 (t, J=6Hz, 2H), 5.08 (d, J=4.5Hz, 1H), 5.62 (d.d, J=4.5Hz and 9Hz, 1H), 6.27 (s, 1H), 6.82(s, 2H), 6.63-7.13 (m, 1H) and 8.87 (d, J=9Hz, 1H)

Elemental Analysis for $C_{27}H_{37}N_9O_6S_3$

Calcd.: C, 47.70; H, 5.49; N, 18.54 (%)

Found : C, 47.79; H, 5.52; N, 18.54 (%)

1-(Cyclopentanecarbonyloxy)ethyl 7β-[2-(2-aminothiazol-4-yl)acetamido]-3-[[[1-(2-dimethylaminoethyl)-1H-tetrazol-5-yl] thio]methyl] ceph-3-em-4-carboxylate (Compound No. 7)

IR(KBr) $cm^{-1}$: 1775, 1740, 1638

NMR ($d_6$-DMSO) δ: 1.47 and 1.55 (two d, J=6Hz, 3H), 1.03-2.03 (m, 9H), 2.23 (s, 6H), 2.76 (t, J=6Hz, 2H), 3.37 (s, 2H), 3.57 and 3.83 (ABq, J=21Hz, 1H), 4.05 and 4.13 (ABq, J=13.5Hz, 1H) 4.40 (t, J=6Hz, 2H), 5.05 (d, J=4.5Hz, 1H), 5.75 (d.d, J=4.5Hz and 9Hz, 1H), 6.24 (s, 1H), 6.81 (s, 2H), 6.66-7.16 (m, 1H) and 8.72 (d, J=9Hz, 1H)

Elemental analysis for $C_{26}H_{35}N_9O_6S_3$

Calcd.: C, 46.90; H, 5.31; N, 18.94 (%)

Found : C, 46.96; H, 5.04; N, 18.77 (%)

1-(Cyclobutanecarbonyloxy)ethyl 7β-[2-(2-aminothiazol-4-yl)acetamido]-3-[[[1-(2-dimethylaminoethyl)-1H-tetrazol-5-yl] thio]methyl] ceph-3-em-4-carboxylate (Compound No. 9)

IR(KBr) $cm^{-1}$: 1775, 1740, 1640

NMR ($d_6$-DMSO) δ: 1.48 and 1.52 (two d, J=6Hz, 3H), 2.26 (s, 6H), 1.62-2.40 (m, 6H), 2.70 (t, J=6Hz, 2H), 2.93-3.27 (m, 1H), 3.35 (s, 2H), 3.63 and 3.83 (ABq, J=18Hz, 2H), 4.07 and 4.15 (ABq, J=13.5Hz, 2H), 4.38 (t, J=6Hz, 2H), 5.06 (d, J=4.5Hz, 1H),

5.71 (d.d, J=4.5Hz and 9Hz, 1H), 6.25 (s, 1H), 6.84 (s, 2H), 6.70-7.12 (m, 1H) and 8.65 (d, J=9Hz, 1H)

Elemental analysis for $C_{25}H_{33}N_9O_6S_3$

Calcd.: C, 46.07; H, 5.11; N, 19.37 (%)

Found : C, 46.21; H, 4.92; N, 19.15 (%)

1-(Cyclopropanecarbonyloxy)ethyl 7β-[2-(2-aminothiazol-4-yl)acetamido]-3-[[[1-(2-dimethylaminoethyl)-1H-tetrazol-5-yl] thio]methyl] ceph-3-em-4-carboxylate (Compound No. 11)

IR(KBr) $cm^{-1}$: 1780, 1738, 1670

NMR ($d_6$-DMSO)δ: 0.72-1.10 (m, 4H), 1.47 and 1.49 (two d, J=6Hz, 3H), 1.43-1.90 (m, 1H), 2.30 (s, 6H), 2.91 (t, J=6Hz, 2H), 3.37 (s, 2H), 3.63 and 3.86 (ABq, J=18Hz, 2H), 3.98 and 4.12 (ABq, J=13.5Hz, 2H), 4.36 (t, J=6Hz, 2H), 5.07 (d, J=4.5Hz, 1H), 5.72 (d.d, J=4.5Hz and 9Hz, 1H), 6.24 (s, 1H), 6.63-7.07 (m, 3H) and 8.86 (d, J=9Hz, 1H)

Example 2

1-(Cyclohexylacetoxy)ethyl 7β-[2-(2-aminothiazol-4-yl)acetamido]-3-[[[1-(2-dimethylaminoethyl)-1H-tetrazol-5-yl]thio]methyl] ceph-3-em-4-carboxylate dihydrochloride (Compound No. 2)

In 20 ml of dichloromethane is dissolved 1.6 g of Compound No. 1, prepared in Example 1, and 2 ml of anhydrous 4.9 N-hydrochloric acid in ethyl ether is added to the solution under ice cooling. The resulting white solid precipitates are collected by filtration, washed with anhydrous ethyl ether and dried under reduced pressure

to give 1.55 g of the above-identified compound as an amorphous solid.

IR(KBr) $cm^{-1}$: 1782, 1750, 1670

NMR ($DCl-D_2O$) δ: 1.56-2.27 (m, 14H), 2.44 and 2.47 (two d, J=5Hz, 2H), 3.28 (s, 6H), 4.04 (s, 2H), 3.86-4.30 (m, 4H), 4.33-4.87 (m, 2H), 5.63 (d, J=4.5Hz, 1H), 5.87 (d, J=4.5Hz, 1H), 6.96 (s, 1H) and 7.17 (q, J=6Hz, 1H)

Elemental analysis for $C_{28}H_{39}N_9O_6S_3 \cdot 2HCl \cdot H_2O$

Calcd.: C, 42.85; H, 5.52; N, 16.06 (%)

Found : C, 42.87; H, 5.91; N, 15.69 (%)

The compounds produced by the same procedure as Example 2 and their physico-chemical constants are shown below.

1-(Cyclohexanecarbonyloxy)ethyl 7β-[2-(2-aminothiazol-4-yl)acetamido]-3-[[[1-(2-dimethylaminoethyl)-1H-tetrazol-5-yl]thio]methyl] ceph-3-em-4-carboxylate dihydrochloride (Compound No. 4)

IR(KBr) $cm^{-1}$: 1775, 1740, 1635

NMR ($DCl-D_2O$) δ: 1.73 (d, J=6Hz, 3H), 1.33-2.70 (m, 11H), 3.28 (s, 6H), 4.02 (s, 2H), 3.92-4.30 (m, 4H), 4.54 (b-s, 2H), 5.33 (d, J=4.5Hz, 1H), 5.87 (d, J=4.5Hz, 1H), 6.93 (s, 1H) and 7.17 (q, J=6Hz, 1H)

Elemental analysis for $C_{27}H_{37}N_9O_6S_3 \cdot 2HCl \cdot H_2O$

Calcd.: C, 42.07; H, 5.36; N, 16.36 (%)

Found : C, 42.39; H, 5.77; N, 16.15 (%)

1-(Cyclopentylacetoxy)ethyl 7β-[2-(2-aminothiazol-4-yl)acetamido]-3-[[[1-(2-dimethylaminoethyl)-1H-tetrazol-5-yl]thio]methyl] ceph-3-em-4-carboxylate dihydrochloride (Compound No. 6)

IR(KBr) $cm^{-1}$: 1772, 1750, 1670

NMR ($D_2O$) δ: 1.53-2.17 (m, 12H), 2.57 (d, J=6Hz, 2H), 3.28 (s, 6H), 4.01 (s, 2H), 3.92-4.23 (m, 4H), 4.55 (b-s, 2H), 5.14 (t, J=6Hz, 2H), 5.39 (d, J=4.5Hz, 2H), 5.91 (d, J=4.5Hz, 1H), 6.83 (s, 1H) and 7.17 (q, J=6Hz, 1H)

Elemental analysis for $C_{27}H_{37}N_9O_6S_3 \cdot 2HCl \cdot H_2O$

Calcd.: C, 42.85; H, 5.52; N, 16.06 (%)

Found : C, 42.87; H, 5.91; N, 15.69 (%)

1-(Cyclopentanecarbonyloxy)ethyl 7β-[2-(2-aminothiazol-4-yl)acetamido]-3-[[[1-(2-dimethylaminoethyl)-1H-tetrazol-5-yl]thio]methyl] ceph-3-em-4-carboxylate dihydrochloride (Compound No. 8)

IR (KBr) $cm^{-1}$: 1772, 1752, 1740, 1670

NMR ($D_2O$) δ: 1.73 (d, J=6Hz, 3H), 1.51-2.43 (m, 9H), 3.28 (s, 6H), 4.02 (s, 2H), 3.92-4.22 (m, 4H), 4.54 (b-s, 2H), 5.11 (t, J=6Hz, 2H), 5.38 (d, J=4.5Hz), 5.91 (d, J=4.5Hz), 6.95 (s, 1H) and 7.16 (q, J=6Hz, 1H)

Elemental analysis for $C_{26}H_{35}N_9O_6S_3 \cdot 2HCl \cdot 1.5H_2O$

Calcd.: C, 41.59; H, 5.43; N, 16.17 (%)

Found : C, 41.42; H, 5.43; N, 15.91 (%)

1-(Cyclobutanecarbonyloxy)ethyl 7β-[2-(2-aminothiazol-4-yl)acetamido]-3-[[[1-(2-dimethylaminoethyl)-1H-tetrazol-5-yl] thio]methyl] ceph-3-em-4-carboxylate dihydrochloride (Compound No. 10)

IR(KBr) $cm^{-1}$: 1780, 1740, 1670

NMR ($D_2O$) δ: 1.73 (d, J=6Hz, 3H), 1.88-2.67 (m, 7H), 3.33 (s, 6H), 4.02 (s, 2H), 3.91-4.22 (m, 4H), 4.53 (b-s, 2H), 5.16 (t, J=6Hz, 2H), 5.39 (d, J=4.5Hz, 1H), 5.92 (d, J=4.5Hz, 1H), 6.94 (s, 1H) and 7.16 (q, J=6Hz, 1H)

Elemental analysis for $C_{25}H_{33}N_9O_6S_3 \cdot 2HCl \cdot 1.5H_2O$

Calcd.: C, 40.20; H, 5.32; N, 16.31 (%)

Found : C, 39.95; H, 5.10; N, 16.77 (%)

1-(Cyclopropanecarbonyloxy)ethyl 7β-[2-(2-aminothiazol-4-yl)acetamido]-3-[[[1-(2-dimethylaminoethyl)-1H-tetrazol-5-yl]thio]methyl] ceph-3-em-4-carboxylate dihydrochloride (Compound No. 12)

IR (KBr) $cm^{-1}$: 1780, 1738, 1670

NMR ($D_2O$) δ: 1.13-1.30 (m, 4H), 1.85 (d, J=6Hz, 3H), 1.89-2.13 (m, 1H), 3.10 (s, 6H), 4.03 (s, 2H), 3.94-4.23 (m, 4H), 4.54 (b-s, 2H), 5.17 (t, J=6Hz, 2H), 5.41 (d, J=5.7Hz, 1H), 5.94 (d, J=5.7Hz, 1H), 6.96 (s, 1H) and 7.18 (q, J=6Hz, 1H)

Elemental analysis for $C_{24}H_{31}N_9O_6S_3 \cdot 2HCl \cdot 2H_2O$

Calcd.: C, 38.61; H, 4.99; N, 16.88 (%)

Found : C, 38.70; H, 4.79; N, 16.80 (%)

Example. 3

Preparation of 1-(cyclohexanecarbonyloxy)ethyl 7β-[2-(2-aminothiazol-4-yl)acetamido]-3-[[[1-(2-dimethylaminoethyl]-1H-tetrazol-5-yl]thio]methyl]ceph-3-em-4-carboxylate dihydrochloride:

Step A: Preparation of 1-(cyclohexanecarbonyloxy)ethyl 7β-amino-3-[[[1-(2-dimethylaminoethyl)-1H-tetrazol-5-yl]thio]methyl]ceph-3-em-4-carboxylate dihydrochloride:

To a solution of 4.52 g of 7β-amino-3-[[[1-(2-dimethylaminoethyl)-1H-tetrazol-5-yl]thio]methyl]ceph-3-em-carboxylic acid dihydrochloride in 60 ml of dimethylformamide, 1.67 g of potassium acetate is added and the mixture is cooled to 0°C with stirring. To the mixture, 4.7 g of 1-iodoethyl cyclohexanecarboxylate is added dropwise, and the mixture is stirred at 0°C for 5 minutes. The reaction mixture is then poured into a mixture of 60 ml of dichloromethane and 60 ml of 0.1N HCl, and the aqueous layer is separated. The aqueous layer is adjusted to pH 6.0 with a saturated aqueous solution of sodium hydrogen-carbonate and extracted with dichloromethane. The organic layer is admixed with water and adjusted to pH 2.0 with 4N HCl. The aqueous layer is separated and dichloromethane is removed from the aqueous layer under reduced pressure. The aqueous layer is lyophilized to give 2.65 g of the titled compound.

IR(Nujol) $cm^{-1}$: 1785, 1755, 1675

Step B: To a mixture of 30 ml of water and 30 ml of dichloromethane is added 1.73 g of the compound prepared in the above step A. While the mixture is stirred, 0.56 g of sodium hydrogencarbonate is added. The organic layer is separated and dried over anhydrous calcium chloride. After the calcium chloride is filtered

off, a solution of 0.61 g of (2-aminothiazol-4-yl)acetic acid hydrochloride and 0.64 g of dicyclohexylcarbodiimide in 20 ml of dimethylformamide is added to the organic layer and the mixture is stirred at room temperature.

After the precipitates are removed by filtration, 150 ml of ethyl acetate and 100 ml of ice-cooled water are added to the filtrate. The organic layer is separated and washed with water and a saturated aqueous solution of sodium chloride, dried over anhydrous magnesium sulfate. After the magnesium sulfate is filtered off, the filtrate is condensed to 10 ml under reduced pressure. To the residual solution is added anhydrous etheral hydrogen chloride. The precipitated white powdery product (0.31 g) is collected by filtration.

IR(KBr) $cm^{-1}$: 1775, 1740, 1635
NMR ($DCl-D_2O$) δ: 1.73 (d, J=6Hz,3H), 1.33-2.70 (m, 11H), 3.28 (s, 6H), 4.02 (s, 2H), 3.92-4.30 (m, 4H), 4.54 (b-s, 2H), 5.33 (d, J=4.5Hz, 1H), 5.87 (d, J=4.5Hz, 1H), 6.93 (s, 1H), 7.17 (q, J=6Hz, 1H)

Example 4

Preparation of 1-(cyclohexanecarbonyloxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate dihydrochloride:

To a mixture of 15 ml of water and 15 ml of dichloromethane is added 1.1 g of 1-(cylohexanecarbonyloxy)ethyl 7β-amino-3-[[[1-(2-dimethylaminoethyl)-1H-tetrazol-5-yl]thio]methyl]ceph-3-em-4-carboxylate dihydrochloride, followed by adding 0.31 g of sodium hydrogencarbonate. After the mixture is stirred, the

organic layer is separated, and dried over anhydrous calcium chloride. After the solvent is removed under reduced pressure, the residue is dissolved in 15 ml of dichloromethane and the solution is cooled to -25°C. To this solution is added a solution of 0.52 g of ω-chloroacetoacetyl chloride in 2.0 ml of dichloromethane. The mixture is stirred at -20 to -15°C for 20 minutes. A solution of 0.76 g of thiourea in 5 ml of dimethylacetamide is added to the mixture, which is then stirred at room temperature for 3 hours. Water is added to the reaction mixture and the aqueous layer is separated, adjusted to pH 6.0 and extracted with dichloromethane. Water is added to the organic layer, which is then adjusted to pH 1.5 with 2N-HCl. The aqueous layer is separated and dichloromethane is removed from it under reduced pressure. The aqueous layer is subjected to the column chromatography on Amberlite XAD-II (produced by Rhom & Haas, USA, insoluble cross-linked polystyrol in bead form) with 120 ml of 0.01 N HCl followed by adding 10% acetonitrile-0.01N HCl as eluents. The eluate is lyophilized to give 0.51 g of white powder.

IR(KBr) $cm^{-1}$: 1775, 1740, 1635

NMR ($DCl-D_2O$) δ: 1.73 (d, J=6Hz, 3H), 1.33-2.70 (m, 11H), 3.28 (s, 6H), 4.02 (s, 2H), 3.92-4.30 (m, 4H), 4.54 (b-s, 2H), 5.33 (d, J=4.5Hz, 1H), 5.37 (d, J=4.5Hz, 1H), 6.93 (s, 1H), 7.17 (q, J=6Hz, 1H)

Example 5

Preparation of 1-(cyclohexanecarbonyloxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate.

a) Preparation of 1-(cyclohexanecarbonyloxy)ethyl 7β-[2-(2-aminothiazol-4-yl)acetamido]-3-acetoacetoxymethyl-ceph-3-em-4-carboxylate.

In 30 ml of N,N-dimethyl formamide, 5.23 g of sodium 7β-[2-(2-aminothiazol-4-yl)acetamido]-3-acetoacetoxymethyl-ceph-3-em-4-carboxylate is dissolved. After the solution is cooled to -5°C, 4.7 g of 1-iodoethyl cyclohexanecarboxylate is added dropwise under stirring and the stirring is continued for 5 min. thereafter. Subsequently, the resulting crude product is treated in the same way as in Example 1 to yield 3.6 g of the titled product a).

IR(KBr) $cm^{-1}$: 1780, 1750, 1675
NMR ($d_6$-DMSO) δ: 1.42 and 1.48 (two d, J=6Hz, 3H), 0.9-2.05 (m, 10H), 2.15 (s, 3H), 3.42 (b-s, 2H), 3.45 (s, 2H), 3.58 and 3.83 (ABq, J=18.5Hz, 2H), 4.00 and 4.18 (ABq, J=13.5Hz, 2H), 5.02 (d, J=4.5Hz, 1H), 5.6-5.8 (m, 1H), 6.23 (s, 1H), 6.8-7.1 (m, 1H), 8.90 (d, J=9Hz, 1H)

b) Preparation of 1-(cyclohexanecarbonyloxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate.

In 30 ml of acetone, 2.5 g of the compound obtained in the foregoing step a) is dissolved. To this solution is added 10 ml of an aqueous solution of 0.9 g of 1-(2-dimethylaminoethyl)-5-mercapto-1H-tetrazole and 0.8 g of sodium bicarbonate and then warmed up to 40°C under stirring for 1 hour. Thereafter, the reaction mixture is poured into a mixture of ethyl acetate (150 ml) and ice water (50 ml) and the organic layer is separated.

The organic layer is washed successively with ice water (50 ml) and saturated aqueous solution of sodium chloride (50 ml) and dried over anhydrous magnesium sulfate. After removing of magnesium sulfate by filtration, the filtrate is concentrated in vacuo. The residue is dissolved in acetone and insolubles are filtered off. To the filtrate is added isopropyl ether until powdery precipitates are deposited. The precipitates are obtained at white powdery product. The yield is 0.14 g.

IR(KBr) $cm^{-1}$: 1775, 1740, 1635

NMR ($DCl-D_2O$)δ: 1.73 (d, J=6Hz, 3H), 1.33-2.70 (m, 11H), 3.28 (s, 6H), 4.02 (s, 2H), 3.92-4.30 (m, 4H), 4.54 (b-s, 2H), 5.33 (d, J=4.5Hz, 1H), 5.87 (d, J=4.5Hz, 1H), 6.93 (s, 1H), 7.17 (q, J=6Hz, 1H)

Example 6

Compound No. 1 according to Example 1 (334.4 g; 250 g as the non-ester compound) is evenly admixed with 70.5 g of hydroxypropylcellulose and 70.5 g of carboxymethylcellulose. The resulting mixture is filled into capsules in amounts of 237.7 mg (125 mg as the non-ester) by the established pharmaceutical procedure.

Example 7

Compound No. 2 according to Example 2 (373.4 g; 250 g as the non-ester compound) is evenly admixed with 70 g of starch and 6 g of hydroxypropylcellulose. The mixture is pressed into tablets by the established pharmaceutical procedure to provide tablets each containing 224.7 mg (125 mg as the non-ester).

Test Example

The compounds produced according to the Examples (Compound Nos. 2, 4, 6 and 8) and, as a reference control,

pivaloyloxymethyl ester of compound (II) (hereinafter referred to as Compound A) are orally administered to mice at a common dose level of 100 mg/kg (as the non-ester, i.e. compound (II) per mouse). After administration, the plasma concentrations of compound (II) at 0.25, 0.5, 1.0 and 2.0 hours are determined by the cup assay method (using Proteus mirabilis Eb 313 as the test organism) and the area under plasma concentration-time curve (AUC) over 0 to 2 hours is calculated.

As a control, compound (II) is subcutaneously administered to mice at the same dose and the AUC is similarly calculated. Bioavailability is calculated by means of the following equation.

$$\text{Bioavailability}(\%) = \frac{\text{AUC (oral administration)}}{\text{AUC (subcutaneous administration)}} \times 100$$

The results are shown in Table 1.

Table 1

| Compound No. | Plasma level of non-ester (μg/ml), n=4* | | | | AUC | Bio-availability |
|---|---|---|---|---|---|---|
| | 0.25 | 0.5 | 1 | 2 hr | (μg.hr/ml) | |
| 2 | 43.9 | 45.2 | 16.4 | 3.1 | 41.8 | 107.7 |
| 4 | 43.6 | 31.7 | 8.38 | 1.01 | 29.6 | 76.2 |
| 6 | 23.2 | 27.2 | 16.9 | 6.46 | 31.9 | 82.2 |
| 8 | 42.5 | 29.9 | 10.5 | 1.35 | 30.4 | 78.3 |
| A | 21.0 | 16.2 | 6.1 | 0.6 | 16.2 | 41.8 |
| Control compound (II) | 69.2 | 29.0 | 13.2 | 1.5 | 38.8 | 100 |

* Average for 4 mice.

0109294

What is claimed is:

1. A compound of the formula:

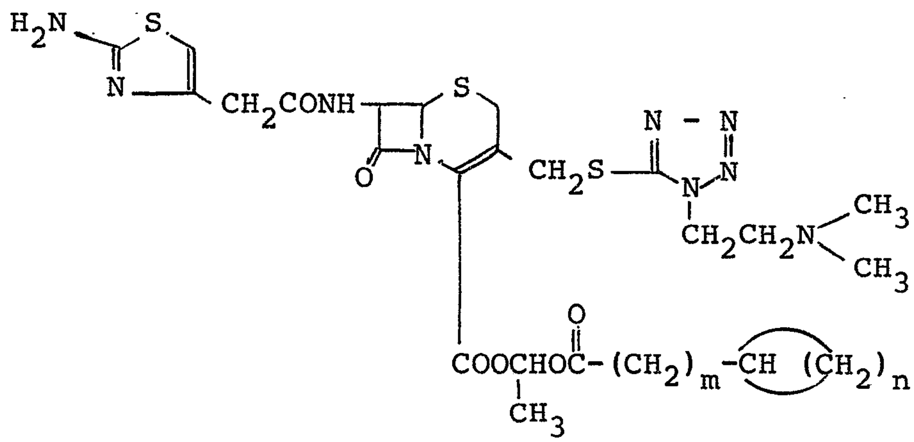

wherein m is an integer of 0 or 1; and n is an integer of 2 to 5, or its pharmaceutically acceptable salt.

2. A compound according to Claim 1, wherein n is an integer of 4 or 5.

3. A compound according to Claim 1, wherein the pharmaceutically acceptable salt is hydrochloride.

4. A compound according to Claim 1, the compound of the formula being 1-(cyclohexylacetoxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate.

5. A compound according to Claim 1, the compound of the formula being 1-(cyclohexanecarbonyloxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate.

6. A compound according to Claim 1, the compound of the formula being 1-(cyclopentylacetoxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate.

7. A compound according to Claim 1, the compound of the formula being 1-(cyclopentanecarbonyloxy)ethyl 7β-[2-(2-aminothiazol-4-yl)acetamido]-3-[[[1-(2-dimethylaminoethyl)-1H-tetrazol-5-yl]thio]methyl]ceph-3-em-4-carboxylate.

8. A process for producing a compound of the formula:

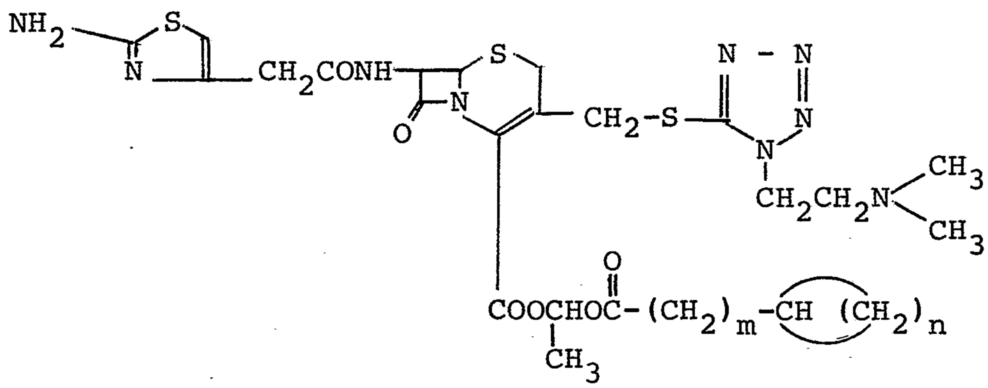

wherein m is an integer of 0 or 1; and n is an integer of 2 to 5, or its pharmaceutically acceptable salt, which comprises (1) reacting a compound of the formula:

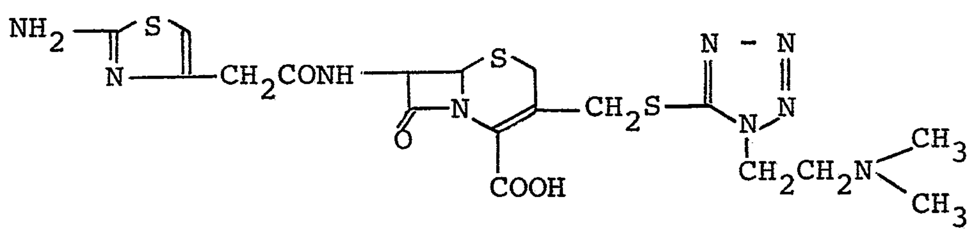

or its salt, with a compound of the formula:

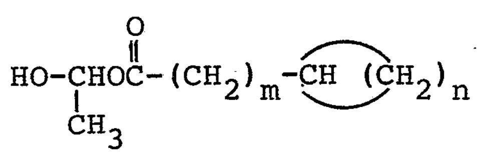

wherein m and n have the same meaning as defined above, or its reactive derivative; or (2) reacting a compound of the formula:

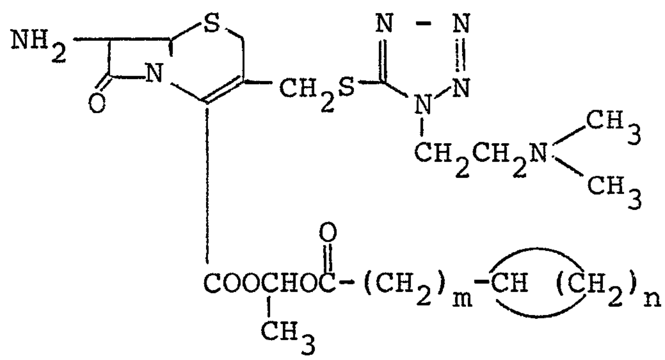

wherein m and n have the same meaning as defined above, with 2-(2-aminothiazol-4-yl)acetic acid or its reactive derivative; or (3) reacting a compound of the formula:

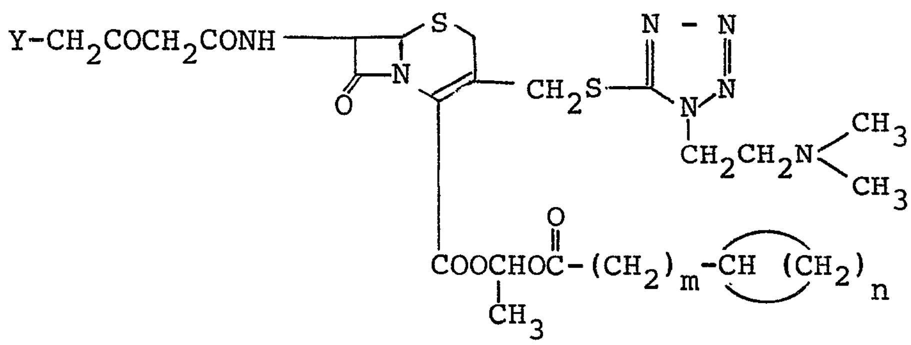

wherein Y is a halogen; m and n have the same meaning as defined above, with thiourea; or (4) reacting a compound of the formula:

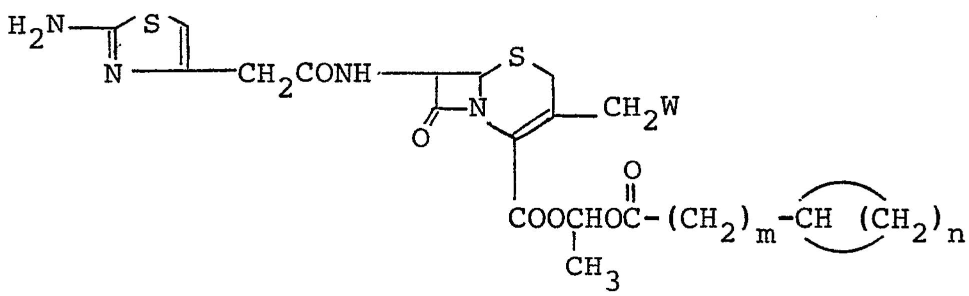

wherein W is acetoxy or acetoacetoxy; m and n have the same meaning as defined above, with 1-(2-dimethylaminoethyl)-5-mercapto-1H-tetrazole.

9. A pharmaceutical composition comprising a compound of the formula:

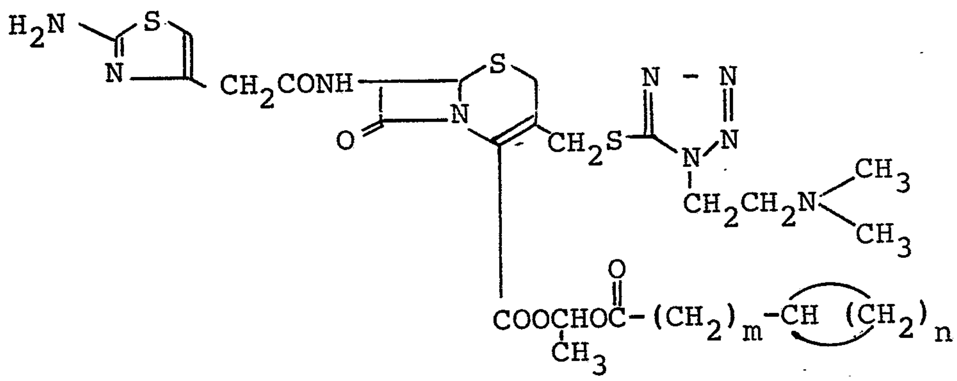

wherein m is in integer of 0 or 1; and n is an integer of 2 to 5, or its pharmaceutically acceptable salt as an effective ingredient.